(19)

**Europäisches Patentamt**
**European Patent Office**
**Office européen des brevets**

(11) **EP 4 552 724 A1**

(12) **EUROPEAN PATENT APPLICATION**

(43) Date of publication:
14.05.2025 Bulletin 2025/20

(21) Application number: 24206248.7

(22) Date of filing: 11.10.2024

(51) International Patent Classification (IPC):
**B01D 53/04** (2006.01)    **C07C 7/12** (2006.01)
**C07C 11/24** (2006.01)

(52) Cooperative Patent Classification (CPC):
(C-Sets available)
**B01D 53/0446; B01D 53/0407; B01D 53/0454;**
**C07C 7/12;** B01D 2256/24; B01D 2257/702;
B01D 2258/025; B01D 2259/40007;
B01D 2259/40009                    (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 08.11.2023 JP 2023190493

(71) Applicant: L'AIR LIQUIDE, SOCIETE ANONYME
POUR L'ETUDE ET
L'EXPLOITATION DES PROCEDES GEORGES
CLAUDE
75007 Paris (FR)

(72) Inventors:
• LAVENN, Christophe
78354 Les Loges-En-Josas (FR)
• OGAWA, Tomofumi
Yokosuka, 239-0847 (JP)
• PROST, Laurent
60439 Frankfurt am Main (DE)
• BENZAQUI, Marvin
78354 Les Loges-En-Josas (FR)

(74) Representative: Air Liquide
L'Air Liquide S.A.
Direction de la Propriété Intellectuelle
75, Quai d'Orsay
75321 Paris Cedex 07 (FR)

(54) **SYSTEM FOR REMOVING RESIDUAL SOLVENT IN ACETYLENE GAS**

(57)    To provide a solvent removal system that removes solvent impurities in gas.

The removal system 1 includes: a gas source unit 11 that includes a storage container; a purifier unit 121 that removes residual solvent, which is organic solvent, from acetylene gas supplied from the gas source unit 11 and that has not been subject to purification or processing; a flow split 123 that distributes the acetylene gas supplied from the gas source unit 11; flow mix means 124 for mixing purified acetylene gas processed in the purifier unit 121 and unpurified acetylene gas supplied from the gas source unit 11; and a control unit CU that determines the purity of the acetylene gas delivered from the flow mix means (124) and controls the flow split (123) and the flow mix means (124) so that the acetylene gas delivered to a demand point PO has a target purity.

[Fig. 1]

**(Cont. next page)**

EP 4 552 724 A1

(52) Cooperative Patent Classification (CPC): (Cont.)

C-Sets
**C07C 7/12, C07C 11/24**

## Description

### Technical Field

The present invention relates to a system for removing residual solvent in acetylene gas. More specifically, the present invention relates to a system for removing minute quantities of residual solvent in acetylene gas released from a storage container.

### Background Art

In low-pressure vacuum carburizing technology, heat treatment under low pressure and vacuum is used for modifying the surface of metal components produced from iron, steel, and other metals and alloys. Also, the low-pressure vacuum carburizing method generates less soot compared with other carburizing methods (for example, the high-concentration carburizing method, the gas carburizing method), so it is gaining attention (for example, see Patent Document 1).

In carburizing technology, acetylene ($C_2H_2$) for example is most widely used as the carbon source (for example, see Patent Document 2).

In the low-pressure vacuum carburizing process, it is necessary to introduce acetylene into a chamber at a constant flow rate. However, in contrast to other hydrocarbon gases, acetylene cannot be safely compressed to a pressure higher than 1. 5 barg (gauge pressure). Therefore, acetylene dissolved in an organic solvent (for example, acetone or dimethylformamide (DMF)) is impregnated into a porous material (for example, a porous calcium silicate material) as a storage technology for acetylene.

When recovering the acetylene in the organic solvent from the porous material, inevitably there is a certain level of organic solvent in the acetylene gas.

In the carburization process, a plurality of storage containers (for example, cylinders) storing acetylene is installed, and it is necessary to control the quantity of acetylene supplied to a chamber in accordance with the process requirements.

Organic solvent (referred to as "residual solvent") at a certain level is contained in the acetylene gas released from the storage container. The concentration of organic solvent in the acetylene gas depends on the nature and temperature of the solvent, the residual pressure in the cylinder, and the speed of release of the acetylene gas (for example, see Patent Document 3)

For example, when the organic solvent is dimethylformamide (DMF), the concentration of the organic solvent in the acetylene gas is in the range of about 0. 01% (100 ppm) to about 1% (10,000 ppm). Also, when the organic solvent is acetone, the range is from about 1% to about 10%.

In the carburization process, there is a possibility that the presence of organic solvent will have an adverse effect on the homogeneity and the quality, and, there is a possibility that maintenance, etc., will be adversely affected. Therefore, there is a requirement to control the concentration of the organic solvent in the acetylene so that it does not exceed a specified concentration.

On the other hand, organic solvent is necessary for storing acetylene in storage containers, and when the acetylene gas is released from the storage container organic solvent is released with it, and in addition it is difficult to release the total amount of acetylene from the storage container.

Also, the concentration of dimethylformamide (DMF) in acetylene gas is a factor of about 10 to 100 times lower than for acetone. Therefore, preferably dimethylformamide (DMF) is used rather than acetone (for example, see Patent Document 4).

On the other hand, in certain regions there are legal restrictions on DMF, and its use is restricted by safety guidelines, etc. Therefore in some cases it is recommended that acetone is used as the organic solvent.

Methods of removing residual solvent from acetylene gas include, for example, a cold trap (for example, Patent Document 5) or a solvent removal device using adsorbent (for example, see Patent Documents 3 and 6). Such solvent removal devices (referred to as a purifier) must be strictly monitored, and it is necessary to limit the flow rate of the acetylene gas introduced. In addition, the content of organic solvent in acetylene varies with time, or there is a possibility that it varies during use. In order words, it is difficult to appropriately set the size, installation conditions, operating conditions, etc., of solvent removal devices (purifiers). Therefore, when using acetylene gas (containing organic solvent) for uses such as a carburizing process, it is necessary to determine the appropriate size of the solvent removal device (purifier), and carry out appropriate maintenance (installation conditions, operating conditions, etc.).

When releasing acetylene gas from a storage container (for example, a cylinder), the residual pressure within the storage container gradually reduces, and this means that the acetylene content within the storage container has reduced. Conventionally, only less than 70% of the acetylene gas in a storage container was released, in order that the concentration of the residual solvent did not increase.

Also, as a result of use, the residual pressure within the storage container is reduced, so the flow rate of the acetylene gas released from the storage container is also reduced. Therefore, it is not possible to completely use all the acetylene within a storage container, so the usage percentage is reduced.

## Prior Art Literature

### Patent Documents

[Patent Document 1] US Patent No. 5702540
[Patent Document 2] US Patent Publication 2003/0168125
[Patent Document 3] International Patent Publication WO2008120160
[Patent Document 4] US Patent No. 8915992
[Patent Document 5] US Patent No. 8309473
[Patent Document 6] US Patent No. 8398747

## Summary of the Invention

Problems to be Solved by the Invention

The present disclosure provides a system that is capable of appropriately removing minute quantities of residual solvent (solvent traces) in acetylene gas, and supplying acetylene (low-concentration residual solvent) that has been controlled in accordance with the specification conditions for a carburizing process.
Also, the present disclosure provides a system capable of using DMF and acetone as the organic solvent.
Also, the present disclosure provides a system capable of increasing the quantity of acetylene gas supplied from a storage container, and reducing the quantity of residual acetylene in a storage container to less than conventionally and increasing the usage percentage (reducing the residual percentage).
Also, the present disclosure provides a system capable of maintaining at a high level the flow rate of acetylene gas from a storage container.

## Means for Solving the Problems

The system for removing residual solvent in acetylene gas (1) includes:

a gas source unit (11) that stores acetylene dissolved in an organic solvent, that includes at least one storage container (for example, an acetylene storage cylinder);
a purifier unit (121) includes at least one purifier (residual solvent removal device) that removes residual solvent that is the organic solvent from the acetylene gas supplied from the gas source unit (11) that has not been subjected to a purification process;
a flow split (123) that distributes the acetylene gas supplied from the gas source unit (11) (feeding it completely in any one direction, distributing it at a prescribed flow rate);
flow mix means (124) that can mix acetylene gas that has been processed and purified in the purifier unit (121) and acetylene gas that has not been subjected to a purification process supplied from the gas source unit (11); and
a control unit (CU) that determines the purity or solvent content of the acetylene gas delivered from the flow mix means (124) and controls the flow split (123) and the flow mix means (124) so that the acetylene gas delivered to a demand point (PO) has a target purity.

The system for removing residual solvent in acetylene gas (1) may include:

a first pipe (L1) that delivers the unpurified acetylene gas to the flow split (123) from the gas source unit (11);
a first branch pipe (L1a) that delivers unpurified acetylene gas to the purifier unit (121) from the flow split (123);
a second branch pipe (L1b) that delivers purified acetylene gas to the flow mix means (124) from the purifier unit (121);
a bypass pipe (L2) that delivers unpurified acetylene gas to the flow mix means (124) from the flow split (123); and
a lead-out pipe (L3) that delivers acetylene gas from the flow mix means (124) to the demand point (PO) at a downstream stage.

The control unit (CU) may determine the purification ratio (X) based on one of a plurality of data from the acetylene gas concentration supplied from the storage container, the concentration of residual solvent in the acetylene gas, the acetylene gas flow rate, the acetylene gas temperature, and the acetylene gas cumulative supply time.

The control unit (CU) may control the acetylene gas supplied from the gas source unit (11) and executes

(1) proportional purification that purifies in accordance with changes in the purification ratio (X),
(2) fixed purification that purifies to a fixed purification ratio,
(3) complete purification that purifies the complete quantity,
(4) switching so that the total quantity is not purified.

A system for removing residual impurities in gas may include:

a gas source unit that includes at least one storage container;
a purifier unit that includes at least one purifier that removes the residual impurities from unpurified gas supplied from the gas source unit;
a flow split (that distributes gas supplied from the gas source unit;
flow mix means that can mix purified gas that has been processed in the purifier unit and unpurified gas supplied from the gas source unit; and
a control unit that determines the purity or impurity content of the gas delivered from the flow mix means and controls the flow split (and the flow mix means so that the gas delivered to a demand point has a target purity.

The gas may be natural gas (NG) that includes variable impurities such as carbon dioxide, water, and so on, or a mixture of gases that include impurities.

Effects

(1) The present invention enables acetylene gas to be extracted at a constant purity from a plurality of cylinders (storage containers) storing acetylene.
(2) When the organic solvent is DMF, the present invention may be constituted so that acetylene gas is not released from a cylinder in which the residual pressure has reduced below about 5 barg, in order to prevent the solvent content in the acetylene gas from exceeding 500 ppm. In this way, when the residual pressure has reduced, the total number of cylinders used is limited. In the present disclosure, about 70 to 80% of the total acetylene stored can be supplied (conventionally less than about 70% could be used).
(3) The system includes a purifier (solvent removal device), and by executing control such as delivery / non-delivery / partial delivery to the purifier, the concentration (content) of residual solvent in the acetylene can be appropriately controlled.
(4) Also, the system is capable of increasing the quantity of acetylene gas supplied from a storage container, and reducing the quantity of residual acetylene in a storage container to less than conventionally and increasing the usage percentage (reducing the residual percentage).
(5) The flow rate of acetylene gas from a storage container can be maintained at a high level.
(6) Even when acetone is used as the organic solvent, the same quality (concentration of residual solvent) can be maintained as for DMF.
(7) The residual solvent in acetylene gas can be either completely or partially removed, and the acetylene purity can be raised to a specific allowable concentration level (the residual solvent concentration can be reduced to a certain level or lower).

**Brief description of the drawings**

FIG. 1 illustrates the function of the removal system;
FIG. 2 illustrates cases A, B, and C of the purification ratio X;
FIG. 3 illustrates an example of the piping configuration in a purification process and a non-purification process;
FIG. 4 illustrates an example of the relationship between the residual pressure in a storage container and the ideal amount of available acetylene;
FIG. 5 illustrates the relationship between the DMF content and the residual pressure;
FIG. 6 illustrates the relationship between the residual DMF content corresponding to FIG. 5 and the amount of acetylene used; and

FIG. 7 illustrates an example of purification process with a different target solvent contents.

## Description of Embodiments

Several embodiments of the present invention are described below. The following is a description embodiment describing examples of the present invention. The present invention is in no way limited by the following embodiments, and also includes a number of variant modes which may be implemented without altering the essence of the present invention. It should be noted that not all of the configurations described below are essential configurations of the present invention.

Embodiment 1

The following is a description of the basic function of a removal system 1 for removing residual solvent in acetylene gas according to Embodiment 1.

The removal system 1 includes a gas source unit 11, a purifier unit 121, a flow split 123 (referred to as "distribution means"), flow mix means 124, and a control unit CU.

The gas source unit 11 includes at least one storage container. The gas in Embodiment 1 is acetylene gas. The acetylene is dissolved in organic solvent (DMF) and impregnated in a porous material and stored in the storage container.

The purifier unit 121 includes at least one purifier 121a that removes residual solvent, which is the organic solvent, from the acetylene gas supplied from the gas source unit 11 and that has not been subjected to a purification process. The at least one purifier 121a may be configured having an adsorbent column or a condenser system for purification arranged in series or in parallel.

The flow split 123 is means for distributing the acetylene gas supplied from the gas source unit 11 (delivering it completely in any direction, distributing it at a prescribed flow rate). The flow split 123 may be constituted from a three-way valve 123a, a damper mechanism, a flow rate control valve, a proportional flow rate control valve, a solenoid valve, and the like.

The flow mix means 124 is means for mixing together acetylene gas that has been processed and purified in the purifier unit 121 and acetylene gas that has not been subjected to a purification process supplied from the gas source unit 11. The flow mix means 124 is capable of delivering purified acetylene gas only to a downstream stage, capable of delivering acetylene gas that has not been subjected to a purification process only to the downstream stage, and capable of mixing both in a prescribed ratio (purification ratio) and delivering the mixture to the downstream stage.

The flow mix means 124 mixes the acetylene gas that has not been subjected to a purification process and the acetylene gas that has been processed and purified in the purifier unit 121 in a prescribed purification ratio ($X = 0$ to 1). The flow mix means 124 may be constituted with a downstream three-way valve 124a, and it may be constituted from a T-shaped pipe and a gate valve or a flow rate control valve.

The flow mix means 124 may be constituted to include a mixer 124a or a buffer tank. In the mixer 124a or the buffer tank, the gas purified in the purifier 121a and the gas that has not been purified are mixed, and gas with a purification ratio (X) is delivered downstream.

The mixing region of the flow mix means 124 may be constituted from a mixing chamber or a buffer chamber. In the case of a buffer chamber, the purified acetylene gas and the directly delivered unpurified acetylene gas may be introduced at the same time at a controlled flow rate, or introduced in turn into the buffer chamber based on set volume fractions. The mixing region can be equipped with one or a plurality of sampling ports, and the solvent in the acetylene gas and/or other impurities may be measured by an analysis instrument.

The control unit CU calculates the concentration of the gas delivered from the flow mix means (124), and controls the flow split (123) and the flow mix means (124) so that the acetylene gas (unpurified gas, completely purified gas) delivered to a demand point (PO) is a target concentration (for example, the acetylene purity required at the demand point, the concentration of the residual solvent, etc.).

The control unit CU may control the flow split 123 so that acetylene gas is completely delivered in one pipe only, or it may control the flow split 123 so that the acetylene gas is distributed in a prescribed purification ratio ($X = 0$ to 1) to the purifier unit 121 and the flow mix means 124.

The control unit CU may determine the purification ratio (X) based on one or a plurality of data from among the acetylene gas concentration supplied from the storage container, the concentration (content) ($\eta_{source}$) of residual solvent in the acetylene gas, the acetylene gas flow rate, the acetylene gas temperature, and the acetylene gas cumulative supply time.

The control unit CU may also control the flow split 123, the flow mix means 124, and flow rate control valves or gate valves provided on each of the pipes (L1, L1a, L2, L1b, L3, and so on), based on a purification ratio (X).

The control unit CU may determine whether or not to deliver acetylene gas to the purifier unit 121 for purification, and determine the delivery time (total flow of purified acetylene gas) in accordance with the requirements of the carburization process at the demand point PO.

The control unit CU may determine whether or not to deliver to a bypass pipe L2, and determine the delivery time (total flow of unpurified acetylene gas) in accordance with the requirements of the carburization process at the demand point PO.

The control unit CU may determine switching information that includes at least the total flow of purified acetylene gas and the total flow of unpurified acetylene gas, in accordance with the requirements of the carburization process at the demand point PO.

The control unit CU may determine the mixing proportions (mixing proportion per unit time) of the acetylene gas processed in the purifier unit 121 and the acetylene gas delivered via the bypass pipe L2.

The control unit CU may determine the proportions of the purified acetylene gas (purification ratio X) and unpurified gas (1-X), and may further determine the proportions of these mixed in the flow mix means 124.

The control unit CU may monitor changes in status of the purifier unit 121 (for example, in use, in regeneration, on standby) or its status of maintenance, and may control the purifier unit 121.

The control unit CU may determine which purifier (adsorbent column) or plurality of purifiers (adsorbent columns) to use or regenerate from among one or a plurality of purifiers 121a that constitute the purifier unit 121, and may perform the necessary control for use or regeneration.

The control unit CU may include an accumulated quantity calculation unit that calculates the quantity of residual solvent (also referred to as "impurities") accumulated in a purifier (adsorbent column or the like) that has been removed from the acetylene gas by the purifier unit 121; a regeneration and replacement time calculation unit that calculates the regeneration time and/or the replacement time of each purifier 121a of the purifier unit 121 in accordance with the accumulated quantity of the residual solvent calculated by the accumulated quantity calculation unit; and a regeneration control unit that controls regeneration means (not illustrated on the drawings), in accordance with the regeneration time and/or the replacement time of each of the purifiers 121a calculated in the regeneration and replacement time calculation unit 302.

The regeneration means may include a heating device for heating adsorbent within a purifier; regeneration gas supply means for supplying regeneration gas used for regenerating adsorbent via a pipe; and an automatic gate valve provided on the pipe.

The regeneration control unit may control turning the automatic gate valve on and off to switch the passage of gas to the purifier for regeneration or to the purifier for use. The regeneration control unit may control turning the heating device on to heat the adsorbent of the purifier for regeneration. The heating device may be an electric heating jacket provided on the exterior of the wall of a storage container. The regeneration control unit may control commands to regeneration gas supply means, the timing of supplying regeneration gas to piping, and the supply time.

A first pipe L1 is a pipe that delivers unpurified acetylene gas to the flow split 123 from the gas source unit 11. A first branch pipe L1a is a pipe that delivers unpurified acetylene gas to the purifier unit 121 from the flow split 123. A second branch pipe L1b is a pipe that delivers purified acetylene gas to the flow mix means 124 from the purifier unit 121. A bypass pipe L2 is a pipe that delivers unpurified acetylene gas to the flow mix means 124 from the flow split 123. A lead-out pipe L3 is a pipe that delivers acetylene gas from the flow mix means 124 to the demand point PO at a downstream stage.

The storage containers and/or each of the above pipes may include a valve (for example, a gate valve, a flow rate control valve), a flow rate meter for measuring the gas flow rate, a gas pressure gauge for measuring the gas pressure, a gas thermometer for measuring the gas temperature, a gas concentration measuring device for measuring the gas concentration, an impurity concentration measuring device (analysis device for minute quantities of solvent) for measuring the concentration of impurities (solvent) in the gas, and so on. The data measured in each of these may be delivered to the control unit CU with their respective associated measurement times and respective instrument identification information to be stored in a recording unit (not illustrated in the drawings).

The gas source unit 11 or the control unit CU may determine the residual solvent content (concentration) ($\eta_{source}$) in the acetylene gas ($Q_{source}$) released from the storage container, measured by an impurity concentration measuring device (analysis device for minute quantities of solvent).

The upstream three-way valve 123a and the downstream three-way valve 124a may be automatically driven valves, or they may be manual valves.

The upstream three-way valve 123a and the downstream three-way valve 124a may be controlled by control commands from the control unit CU. Each gate valve and flow rate control valve may be controlled by control commands from the control unit CU.

The control unit CU may determine the purification ratio (X) based on one or a plurality of data from among the residual solvent content (concentration) ($\eta_{source}$) in the acetylene gas, the residual pressure of the cylinder final use condition, the acetylene gas flow rate, the acetylene gas temperature, and the cumulative supply time.

The control unit CU controls the flow split 123, the flow mix means 124, and flow rate control valves or gate valves provided on each of the pipes L1, L1a, L2, L1b, L3, and so on, based on the purification ratio (X). The control unit CU controls branching means or each type of valve in accordance with the purification ratio X. The amount of gas delivered to the purifier and the amount of gas delivered to the bypass pipe L2 are controlled.

A gas flow rate meter, gas pressure gauge, gas thermometer, gas concentration measuring instrument, and impurity concentration measuring device may be provided on each of the pipes. The data measured in each of these may be delivered to the control unit CU with their respective associated measurement times and respective instrument identification information, to be stored in a recording unit.

Method of Determining the Limiting Residual Pressure

The control unit CU and/or the gas source unit 11 determines a limiting residual pressure for the storage containers, in accordance with specific conditions of use (flow rate, temperature).

An allowable residual solvent (impurity) content in the acetylene gas is first determined in accordance with the conditions of use, and the residual pressure at which the quality of the target carburization process cannot be obtained is determined.

When the internal pressure measured in a storage container that is in use drops below the limiting residual pressure, use is switched to another storage container. This switching is referred to as the switching point (point of limiting use). This point of limiting use can be used to determine the purification process starting and ending timing of the purifier unit 121.

Also, the switching point can be determined based on data such as the measurement (monitoring) data from each of the measurement devices, or the residual pressure and the temperature within the storage container. The switching point is calculated based on information that can be measured, such as the residual pressure and the temperature of a storage container, the solvent content within the acetylene gas, the cumulative supply time, the total quantity of acetylene gas supplied, the flow rate of the acetylene, and so on.

By determining the switching point, the purification system can be optimized (number of purifiers, size of adsorbent column, etc.).

The concentration of the acetylene gas (residual solvent concentration) released from the container may be measured in advance corresponding to the residual pressure and temperature, from the data an approximation function may be determined, and the approximation function (may be a linear function) may be stored in a storage unit. Using this approximation function, the acetylene gas concentration (residual solvent concentration) can be calculated from the measurement values for the residual pressure and the temperature.

Replacement of the storage container, use of purifiers, use of the bypass pipe, use of the mixing means, and so on, may be determined in accordance with the acetylene concentration and flow rate required at the demand point PO.

Also, it is unnecessary to constantly use purifiers, so the size of the system can be reduced compared with a system in which purifiers are constantly used. By reducing the size of the purifiers, the regeneration energy of the purifiers can also be reduced.

Three types of purification process are performed in accordance with the switching point.

(1) Proportional purification

The amount of gas to be purified and the amount of gas not to be purified are changed and mixed in real time or at specific time intervals (one minute units, 10 minute units, etc.) in accordance with the change in the calculated purification ratio (X). Purification of the acetylene gas is varied with the fixed maximum residual solvent content in the acetylene gas as the upper limit.

(2) Fixed purification

When it is judged that purification is necessary, the purification process is performed in accordance with a fixed purification ratio set in advance.

(3) Complete purification (or non-purification)

When it is judged that purification is necessary, 100% of the acetylene gas is purified (the gas is delivered to the purifier unit, without delivering gas to the bypass pipe). When it is judged that purification is not necessary, 100% of the acetylene gas is delivered to a downstream stage (the gas is delivered to the bypass pipe, without delivering gas to the purifier).

For determining the switching point, the relationship between the residual pressure of the storage container during the period of use and the concentration of the acetylene gas (concentration of the solvent) to be released can be specified by a specific function, based on storage container initial values (volume, filling pressure, and solvent quantity) and the acetylene solubility. Using this function, the ideal acetylene supply that can be used in the downstream stage carburization process can be determined.

(a) A generally used working pressure limit can be determined from the storage container residual pressure.
(b) Depending on the target final use pressure (residual pressure), and depending on the conditions of use (flow rate and temperature), it is possible to achieve an increase of 10% to 60% of the acetylene gas that can be used. For example, in the case of a DMF cylinder (long term use from 5 barg to 2 barg), the quantity of acetylene that can be used is increased from about 20% to 30%, and in the case of an acetylene cylinder (long term use from 6 barg to 2 barg), the quantity of acetylene that can be used is increased from about 35% to 40%.

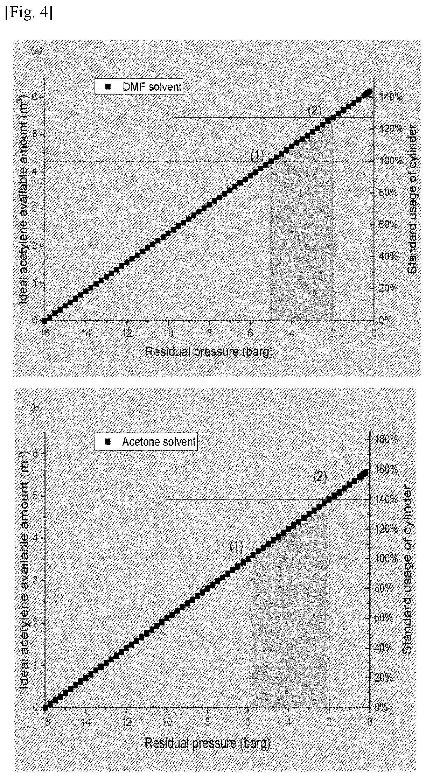

FIG. 4 illustrates the relationship between the residual pressure in a storage container and the ideal available amount of acetylene. Functions for the residual pressure within a storage container (cylinder) at 20°C are illustrated in FIG. 4(a) for a standard B50 cylinder filled with acetylene gas with DMF solvent, and in FIG. 4(b) for a standard B50 cylinder filled with acetylene gas with acetone solvent.

(1) The switching point is determined in accordance with the limiting value of each residual solvent (impurity).

DMF; 500 ppm/5 barg
Acetone: 2.2%/6 barg (flow rate assumed to be 10 slm).

The content of solvent in the acetylene gas is determined based on commonly recommended matters regarding storage container use.
(2) In FIG. 4, the horizontal axis is the residual pressure, the vertical axis on the right is the standard usage percentage of a storage container, and the vertical axis on the left is the ideal amount of acetylene that is available to be used.

The usage percentage is assumed to be 100% for conventional standard use. According to the removal system 1 of the present disclosure, the timing of replacement of a storage container can be extended to the minimum residual pressure (2 barg), and the usage percentages are increased to 130% (DMF FIG. 4(a)), and 140% (acetone FIG. 4(b)), so the amount that can be used is increased.

Determining the target purification ratio (X) is performed based on the estimated solvent content ($\eta_{direct}$) and the user requirement (target solvent content $\eta_{target}$). The control unit CU can determine it by various methods.

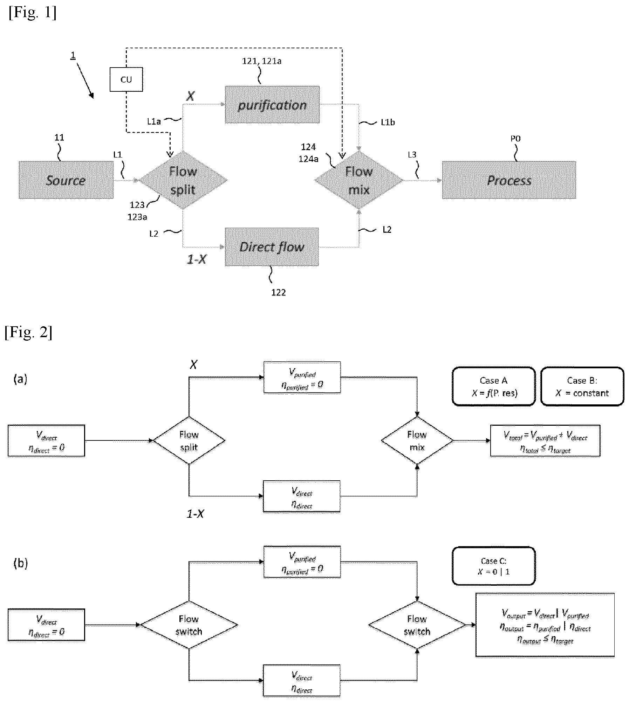

FIG. 2 illustrates two types of approach. Proportional purification (Case A and B) and complete purification (Case C) are illustrated. The main difference between these two approaches is that in proportional purification a portion of the acetylene gas from the storage container is purified (purification ratio X), and in contrast in complete purification all the acetylene gas from the storage container is purified after switching.

(1) Case A: After the switching point is reached, purification is performed to a purification ratio (X) that is varied in accordance with the evaluation result (FIG. 2(a))
(2) Case B: After the switching point is reached, purification is performed to a fixed purification ratio (X) (FIG. 2(a))
(3) Case C: After the switching point is reached, all the acetylene gas is purified (FIG. 2(b))

FIG. 2(a) illustrates proportional purification with a purification ratio (X) in which the acetylene gas is distributed so that only a portion of the acetylene gas is purified. FIG. 2(b) illustrates complete purification in which the acetylene gas from the storage container (cylinder) is completely purified by the purifier unit 121.

The fixed purification ratio (X) may be set to a fixed ratio in accordance with the requirement of the final demand point PO. The variable purification ratio (X) is determined as the target value of the fixed maximum content of the residual solvent in the acetylene gas.

Proportional purification

The control unit CU determines the amount (proportion) of the gas to be purified in the purifier unit 121. Next, the purified gas is mixed with the unpurified gas that has not been subjected to the purification process, and mixed gas is generated. The content of solvent (impurity) in the mixed gas is in accordance with the requirement of the process in the downstream stage.

The advantage of the removal system 1 is that the size of the purifier unit can be reduced compared with a purifier system that purifies the total amount of acetylene gas supplied to the process at the downstream stage.

In this embodiment, the purification ratio X of the purified gas may be a fixed value. The purification ratio may be determined by re-evaluation. The variable purification ratio may be determined by dynamic evaluation.

In another embodiment, the purification ratio X or whether or not to perform complete purification may be determined based on fixed values such as the target final residual pressure (before storage container replacement). In such cases, the purification ratio X is determined as a single value by a predictive method. In addition, a fixed purification ratio X may be estimated based on the number of uses in a specific range, the gas volume, or the residual pressure (can be considered to be intermediate between Cases A and B).

The proportion of gas to be purified (purification ratio X) is determined based on the solvent (impurity) content of the storage container ideally evaluated ($\eta_{source}$), the target solvent (impurity) content ($\eta_{target}$), and the solvent (impurity) content in the purified acetylene gas after purification ($\eta_{purified}$).

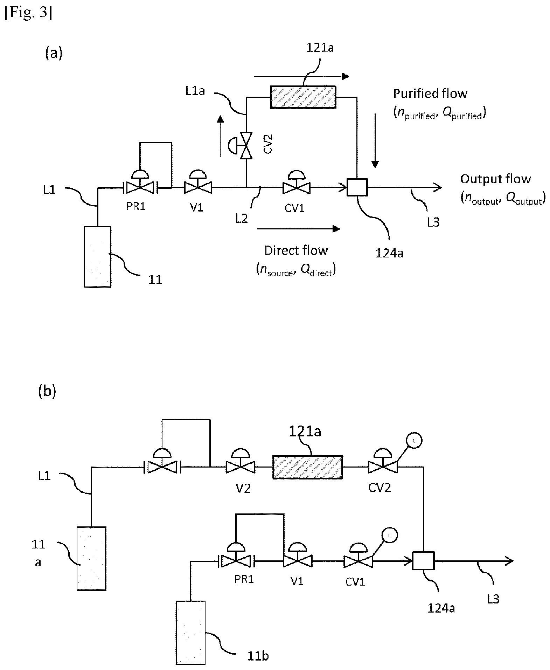

The solvent (impurity) content of the mixed gas after purification can be determined, or the solvent (impurity) content as a result of switching to complete purification is determined. FIG. 3 illustrates examples of the purification processes of the removal system 1. This system can generate acetylene gas of a prescribed purity by purifying all or a portion of the acetylene from the acetylene gas source unit 11 using the purifier unit 121 and mixing it with the unpurified acetylene gas.

The relationship between each solvent concentration and the purification ratio X is shown in Formula (1).

$$(\text{Formula 1}) \qquad \eta_{output} = (1\text{-}X).\eta_{source} + X.\eta_{purified}$$

The relationships for the total gas flow rate $Q_{total}$ (= $Q_{source}$ = $Q_{output}$) are shown in Formula (2), Formula (3), and Formula (4). Here, the state before the switching point corresponds to X = 0.

$$(\text{Formula 2}) \qquad Q_{total} = Q_{purified} + Q_{direct}$$

$$(\text{Formula 3}) \qquad Q_{purified} = X.Q_{total}$$

$$(\text{Formula 4}) \qquad Q_{direct} = (1\text{-}X).Q_{total}$$

Flow rate of acetylene gas from the storage container: $Q_{source}$ (= $Q_{total}$)

Solvent content in the acetylene gas from the storage container: $\eta_{source}$

Flow rate of acetylene gas subjected to the purification process in the purifier: $Q_{purified}$ Solvent content (concentration) in the acetylene gas after the purification process in the purifier: $\eta_{purified}$

Flow rate of acetylene gas that has bypassed the purifier: $Q_{direct}$

Solvent content in the acetylene gas that has bypassed the purifier: $\eta_{direct}$

Flow rate of acetylene gas output from the removal system 1: $Q_{output}$

Solvent content in the acetylene gas output from the removal system 1: $\eta_{output}$

Target solvent content from the user's requirements: $\eta_{target}$

Pressure regulating valve with pressure gauge: PR1

Upstream side flow rate control valve: V1

Flow rate control valve provided on the purifier pipeline L1 (L1a): CV2

Flow rate control valve provided on the bypass pipeline L2: CV1

With CV1 and CV2, the function of the branching means 123 can be realized.

The removal system 1 can dilute the residual solvent (and impurities) in the acetylene gas released from the storage container. (1) The purifier unit 121 may be configured having one or a plurality of columns filled with adsorbent or a solvent condenser. The purifier unit 121 can generate an acetylene gas flow from the acetylene gas released from the storage container with a higher purity (for example, at least 50% removal of the solvent vapour).

(2) The purified acetylene gas and the unpurified acetylene gas can be mixed or the conditions of use can be controlled.

The removal system 1 can enable most of the acetylene gas in the storage container to be used, and on the other hand can reduce the amount of residual solvent in the acetylene gas supplied to the process at the downstream stage.

With the example of a standard DMF solvent storage container with a prescribed (or allowable) DMF content of 500 ppm, conventionally it was necessary to stop use when the residual pressure was 5 barg. Assuming that this was an appropriate stop for a storage container in use, only about 60% to 70% of the total amount of the acetylene gas in the storage container could be used. On the other hand, with the removal system 1, it is possible to operate down to a residual pressure of 2 barg without exceeding the target solvent content $\eta_{target}$, and use 70% to 80% of the total amount of the gas in the storage container. Therefore it is possible to extend the use of the storage containers (cylinders, etc.) on site, and reduce the frequency of replacement and replenishment of storage containers.

The control unit CU can determine the amount to be purified and what state.

Purity of the acetylene in the supplied gas < target purity of the acetylene in the purified gas
Solvent content in the supplied gas > solvent content in the purified gas (mixed gas)

The control unit CU may control each gate valve to completely stop the flow from the gas source unit 11 (storage container) when the quality of the acetylene gas has dropped below the standard value and it is not possible to switch to another storage container.

The control unit CU may estimate the total amount of acetylene gas used based on (i) the conditions of use (residual pressure in the storage container when starting, and residual pressure during use) and (ii) an integral of the flow rate of the acetylene gas delivered to the process at the demand point.

The control unit CU may acquire or determine information regarding the current percentage of use of the storage container (cylinder), the predicted time until the switching point and the remaining time (usable time, replacement time) for replacement of the storage container (cylinder), in accordance with any of the mean values based on the processing time of the carburation process registered in advance, or data on use or registered data.

The control unit CU may output an alarm to the user when a problem occurs due to cooling of the storage container associated with release of the acetylene gas. An alarm may be output to the user under circumstances where the change in the conditions of use are too large (flow rate problem, temperature, residual pressure too low), etc. Also, to support stock control and dispatch by the user, when a storage container reaches a specific usage percentage, this fact may be output.

The control unit CU may have one or a plurality of processors and various types of data and control commands are read from a storage unit of the processor, and the processes necessary for each type of control may be executed.

The control unit CU may exchange each type of data and command signals with an external device. The external device may be, for example, the gas source unit 11, flow split 123, purifier unit 121, flow mix means 124, a memory device, a cloud server, cloud storage, a demand point PO control device, a user controlled device, and so on.

The various types of data can include, for example, the above measured data, determined data, the purification ratio X, the switching point (point of limiting use), the estimated time of replacement of a storage container, the residual pressure, each type of process data, control command data, and so on.

This data may be used to improve management of various aspects, such as stock control, quality control, and so on.

The control unit CU may control and monitor the whole removal system 1. Table 1 shows examples of the cases of purification process.

Residual pressure of the storage pressure during use: $P_{residual}$
Switching point: Sp = limiting residual pressure in normal use. In the present disclosure, the amount of residual solvent is controlled by performing purification at residual pressures lower than this.

[Table 1]

| | | Purification ratio X[a] Re-evaluation ratio | Condition |
|---|---|---|---|
| Case A | | From no purification to purification (X = 0 to 0.9)<br>Proportional purification | $P_{residual}$ > Sp |
| Case B-1 | | There is purification (fixed as desired in the range X = 0.1 to 0.9)<br>Fixed ratio purification<br>Purification within a set time | When there is faulty detection[b] |
| Case B-2 | | There is purification (variable in the range X = 0.1 to 0.9)<br>Fixed ratio purification<br>Executed until the measured value of the residual pressure returns to the normal range | When there is faulty detection[b] |

(continued)

|  | Purification ratio X[a] Re-evaluation ratio | Condition |
|---|---|---|
| Case C | Complete purification (X = 1)<br>No delivery to the bypass pipe<br>Execute purification until $P_{residual}$ is the final residual pressure | $P_{residual} < Sp$ |
| | (a): Purification ratio X: The quantity of purified acetylene gas purified by the purifier unit / total quantity of unpurified acetylene gas released from the storage container<br>(b): Temperature, residual pressure, or other measurement parameter associated with a major change relative to the normal value or estimated value from the control unit. | |

**Embodiments of purification**

The control unit CU may determine the limiting residual pressure before replacement of a storage container and the range of pressure margin (offset value) higher than the limiting residual pressure corresponding to the limiting purity level (lower limit concentration, or the target solvent content $\eta_{target}$) determined in advance for the carburizing process at the demand point.

FIG. 3 illustrates examples of the different purification processes. Proportional purification (Case A), fixed purification (Case B), and complete purification (Case C) are illustrated. In proportional purification, only a portion of the acetylene gas is purified (purification ratio X). In complete purification, all the acetylene gas is purified. The purification process can set the switching point (including offset) as standard.

Proportional purification

Proportional purification purifies a portion of the acetylene gas released from a storage container.

The control unit CU determines the amount of the acetylene gas to be purified by the purifier 121a. The purified acetylene gas is mixed with the unpurified acetylene gas in the flow mix means 124. In this way, the residual solvent content (solvent concentration) in the acetylene gas is reduced. By performing a partial purification process, the size of the purifier unit can be reduced compared with other systems that always purify.

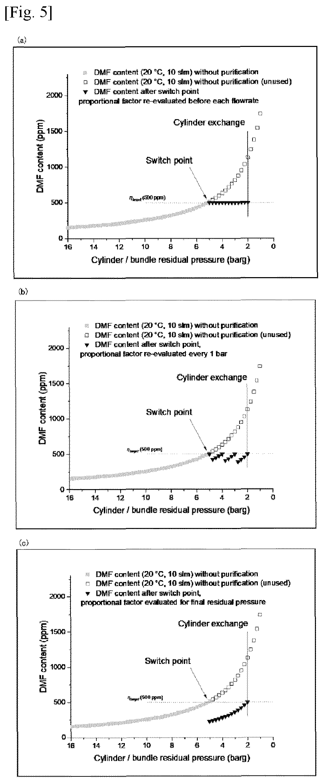

FIG. 5 illustrates the relationship between the DMF content and the residual pressure. The acetylene gas flow rate is 10 slm, and the temperature is 20°C. In the present embodiment, the target solvent content $\eta_{target}$ was set to 500 ppm. It can be seen that as the residual pressure is lowered the DMF content in the acetylene gas increases. Using this relationship curve, the purification ratio X, the proportional purification, the fixed purification, and the complete purification can be set.

In FIG. 5(a) it can be seen that by starting proportional purification at the switching point (switch point: 5 barg), the DMF content in the acetylene gas can be maintained constant (500 ppm) even though the residual pressure reduces from 5 barg to 2 barg. FIG. 5(b) illustrates the result of executing fixed rate purification at each of 5, 4, and 3 barg (the fixed purification rate is increased as the pressure is reduced), so that the DMF content does not exceed 500 ppm. FIG. 5(c) illustrates the result of setting the minimum residual pressure to 2 barg at the timing of replacement of the storage container, and executing complete purification so that at that value the DMF content does not exceed 500 ppm.

The control unit CU may re-evaluate (re-set) the purification ratio X. The re-evaluation (re-set) depends, for example, on the distribution accuracy of the flow split 123.

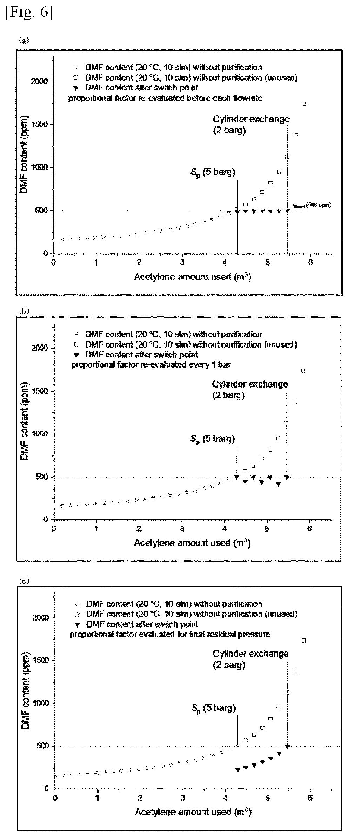

FIG. 6 illustrates the relationship between the residual DMF content corresponding to FIG. 5 and the amount of acetylene gas used.

As the cumulative amount of acetylene gas released from the storage container increases, the residual DMF content in the acetylene gas increases. FIG. 6(a), which corresponds to FIG. 5(a), illustrates that the residual DMF content is the constant value of 500 ppm from the switching point (5 barg) to the final minimum residual pressure (2 barg). Likewise, FIG. 6(b) corresponds to FIG. 5(b), and FIG. 6(c) corresponds to FIG. 5(c).

The quantity of purified acetylene gas delivered from the purifier unit 121 (purification ratio X) and the quantity of unpurified acetylene gas directly delivered to the carburization process at the demand point PO (1-X) are controlled to supply an appropriate total flow rate ($V_{total}$) for one or a plurality of carburization processes. Note that the quantity of purified acetylene gas (purification ratio X) and the quantity of unpurified acetylene gas (1-X) are mixed in the flow mix means 124 (mixer 124a or the like) and delivered to the demand point PO.

Solvent content in the purified acetylene gas: $\eta_{purified}$
Quantity of purified acetylene gas: $V_{purified}$
Quantity of unpurified acetylene gas directly delivered: $V_{direct}$

(Formula 11)

$$V_{total} = V_{purified} + V_{direct}$$

(Formula 12)

$$V_{purified} = X.V_{total}$$

(Formula 13)

$$V_{direct} = (1-X).V_{total}$$

(Formula 14)

$$\eta_{total} = (1-X).\eta_{total} + X.\eta_{purified}$$

The solvent content in the acetylene gas released from the standard storage container (acetylene cylinder) ($\eta_{direct}$) can be appropriately expressed as a function of the residual pressure in the storage container. For example, the function can be a logistics function, or a linear function in the region where the residual pressure is restricted by a fixed temperature and a fixed gas flow rate.

In other words, the solvent content in the acetylene gas extracted from the storage container ($\eta_{direct}$) can be obtained based on data such as the acetylene flow rate, the residual pressure, the temperature, and so on.

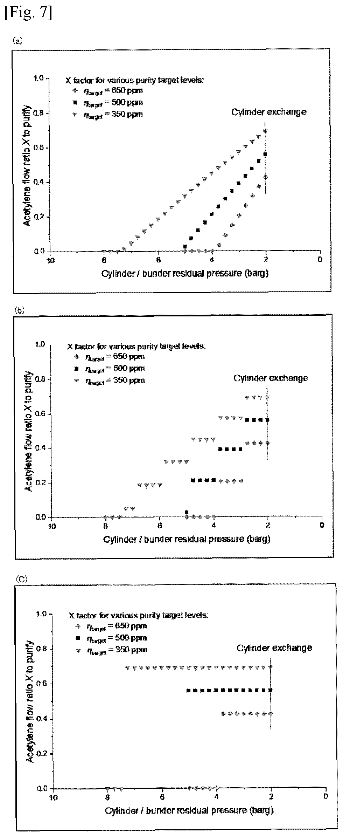

FIG. 7 illustrates the purification process for three types of target solvent content ($\eta_{target}$). The flow rate is 10 slm, and the temperature is 20 °C. In FIG. 7, purification starts from a residual pressure of 7 barg when the target solvent content ($\eta_{target}$) is set to 350 ppm, from a residual pressure of 5 barg when it is set to 500 ppm, and from a residual pressure of 4 barg when it is set to 650 ppm. FIG. 7(a) corresponds to FIG. 5(a), FIG. 7(b) corresponds to FIG. 5(b), and FIG. 7(c) corresponds to FIG. 5(c).

Conditional purification

In conditional purification (FIG. 2(b)), the acetylene gas is either completely purified subject to conditions, or it is not purified.

The conditions are determined by the control unit based on process requirements, gas flow rate, storage container temperature, residual pressure, and other measurement values. The timing for switching the flow switch for executing either complete purification or no purification is determined as a specific value of the residual pressure (for example, 5 barg). In the removal system constituted as illustrated in FIG. 3(a) or 3(b), the purification process is executed.

The acetylene gas delivered from the removal system to the carburization system is either completely purified or not purified.

Before switching: The solvent content in the acetylene gas output is the same as $\eta_{direct}$, $V_{direct}$.
After switching: The solvent content in the acetylene gas output is $\eta_{purified}$, $V_{purified} \doteqdot V_{direct}$.

The control unit CU controls and monitors each type of valve. Such valves are, for example, solenoid valves, pneumatic valves, or pneumatic or solenoid three-way valves.

Purifier unit

The purifier unit 121 includes one or a plurality of purifiers 121a. The purifier 121a may include a first refining device for removing minute quantities of solvent as a liquid, a removal device or purifier for removing minute quantities of solvent such as a mechanical trap or a cold trap (second refining device), or a third refining device for removing other minute impurities (such as sulfur or phosphine compounds, and so on).

The purifier 121a may be constituted from one or a plurality of columns filled with filling material. The filling material may include one or a plurality of, for example, zeolites, exchange zeolites, activated carbon, processed (or impregnated) activated carbon, metal-organic frameworks (MOF), covalent organic frameworks (COF), polymer, silica materials, and so on. The filling material may be in the form of, for example, film, monolith, pellets, beads, blocks, or powder.

The purifier 121a may be constituted from columns arranged in series, columns arranged in parallel, or both of these arrangements of columns.

Formula 15 shows a formula for obtaining the quantity of purified acetylene gas.

$Q_{additional}$: Total quantity of acetylene satisfying the quality.
$Q_{direct}$: Total quantity of acetylene delivered from a storage container
$Q_{purified}$: Quantity of purified acetylene gas purified after the switching point (Sp)
$P_f$: Final minimum residual pressure in the storage container
$q_{C2H2}$: Quantity of acetylene gas delivered in the specified pressure range
$q_{C2H2}$ can be assumed to be the difference in the solubility of the acetylene gas in the quantity of solvent, assuming heat transfer in the ideal time and the usage time.
[Number 1]

Formula 15

$$Q_{additional} = \int_{S_p}^{P_f} q_{C\,2H\,2}\,dP = Q_{direct} + Q_{purified}$$

Formula 16 is a calculation formula for obtaining the quantity of removed solvent. It can be expressed as a function of pressure in the ideal case of constant temperature and constant gas flow rate.

$Q_{solvent\;removed}$: Quantity of solvent removed by the purifier unit
$\eta_{total}$: Total quantity of the solvent content in the acetylene gas delivered to the carburization process
$\eta_{direct}$: Solvent content in the unpurified acetylene gas
[Number 2]

Formula 16

$$Q_{solvent\;removed} = \int_{S_p}^{P_f} (\eta_{direct} - \eta_{total})\,dP$$

The removal system 1 enables the gas source units 11 to be made smaller by a factor of 2 to a factor of 50 compared with a conventional purification system that is constantly purifying the storage containers (gas cylinders or bundles) from the initial pressure to the final pressure. The quantity of adsorbent, the installation area, the number of regenerations, the regeneration energy, the maintenance, and so on, can be reduced.

The purification capacity is defined by the volume of acetylene gas with a given solvent content that can be purified by a certain quantity of adsorbent.

For example, "10% ▪ $m^3$ ▪ $kg^{-1}$ purification capacity H" indicates the property of being able to purify up to 10 $m^3$ of acetylene with 1% solvent content with 1 kg of the adsorbent material M. This is the same as 1 kg of the same adsorbent material being capable of purifying up to 1 $m^3$ of acetylene with 10% solvent content. In the case of two types of substance M1 and M2 having the capacity of purifying the solvent (acetone) in acetylene at the rate 8% ▪ $m^3$ ▪ $kg^{-1}$ and 18% ▪ $m^3$ ▪ $kg^{-1}$ respectively, the size of purifier for a single column for a target purification content of 2.3% can be estimated based on a flow rate of 10 slm at a temperature of 20°C, as shown in Table 2.

Table 2 shows a comparison of the ideal quantity of adsorbent required for complete purification of a B50 gas cylinder modelled for a flow rate of 10 slm at a temperature of 20°C and the corresponding dimensional ratios based on the acetone content (purification capacity H (M1) = 8% ▪ $m^3$ ▪ $kg^{-1}$ and purification capacity H (M2) = 18% ▪ $m^3$ ▪ $kg^{-1}$).

[Table 2]

| | M1 amount (kg) | M2 amount (kg) | Amount of adsorbent relative to full use |
|---|---|---|---|
| Full use (complete purification) | 12.8 | 5.7 | 100% |
| Case A | 0.4 | 0.2 | 3% |
| Case B-1 | 0.5 | 0.2 | 4% |
| Case B-2 | 0.7 | 0.3 | 5% |
| Case C | 1.3 | 0.6 | 10% |

Using the quantities of adsorbent estimated in Table 2, the size reduction of the purifier (adsorbent column, condenser, etc.) can be determined. Cases A to C are compared with full use. The quantity of adsorbent is smallest for proportional purification (Case A) compared with the other cases.

One or more from Case A to C can be applied based on the restriction in solvent content at the demand point determined by the user. Also, the efficiency of the removal system 1 can be increased by selecting each case. A higher usage percentage of acetylene can be achieved, and it is possible to deliver to the demand point acetylene with a quality (purity) that satisfies requirements set in advance. Also, the removal system 1 can reduce the frequency of release of acetylene gas from the storage container, and reduce the cost of supplying acetylene gas.

The control unit CU may record, manage, and transmit the system status and information to one or a plurality of storage devices. The control unit CU may predict the storage container usage percentage and replacement time, maintenance, and the necessity for purifier regeneration. The predicted data may be transmitted to a control centre and used for predicting troubleshooting when necessary.

[Explanation of the Reference Numerals]

| | |
|---|---|
| 1 | Acetylene gas residual solvent removal system |
| 11 | Gas source unit |
| 121 | Purifier unit |
| 121a | Purifier |
| 123 | Flow split |
| 124 | Flow mix means |
| CU | Control unit |

**Claims**

1.  Acetylene gas residual solvent removal system, comprising:

    a gas source unit (11) that stores acetylene dissolved in an organic solvent, and that includes at least one storage container;
    a purifier unit (121) that includes at least one purifier that removes residual solvent that is an organic solvent from the acetylene gas supplied from the gas source unit (11) that has not been subjected to a purification process;
    a flow split (123) that distributes acetylene gas supplied from the gas source unit (11);
    flow mix means (124) that can mix acetylene gas that has been processed and purified in the purifier unit (121) and acetylene gas that has not been subjected to a purification process supplied from the gas source unit (11); and
    a control unit (CU) that determines the purity or solvent content of the acetylene gas delivered from the flow mix means (124) and controls the flow split (123) and the flow mix means (124) so that the acetylene gas delivered to a demand point PO has a target purity.

2.  Removal system according to Claim 1, further comprising: a first pipe (L1) that delivers the unpurified acetylene gas to the flow split (123) from the gas source unit (11);

    a first branch pipe (L1a) that delivers unpurified acetylene gas to the purifier unit (121) from the flow split (123);
    a second branch pipe (L1b) that delivers purified acetylene gas to the flow mix means (124) from the purifier unit (121);
    a bypass pipe (L2) that delivers unpurified acetylene gas to the flow mix means (124) from the flow split (123); and
    a lead-out pipe (L3) that delivers acetylene gas from the flow mix means (124) to the demand point (PO) at a downstream stage.

3.  Removal system according to Claim 1, wherein the control unit calculates a purification ratio (X) based on one or a plurality of data from among the acetylene gas concentration supplied from the storage container, the concentration of residual solvent in the acetylene gas, the residual pressure at the final condition of use of the storage container, the acetylene gas flow rate, the acetylene gas temperature, and the acetylene gas cumulative supply time.

4.  Removal system according to Claim 1, wherein the control unit controls the acetylene gas supplied from the gas source unit (11) and switches

(1) proportional purification that purifies in accordance with changes in the purification ratio (X),
(2) fixed purification that purifies to a fixed purification ratio,
(3) complete purification that purifies the complete quantity,
(4) no purification so that the total quantity is not purified.

5. System for removing residual impurities in gas, comprising:

a gas source unit that includes at least one storage container;
a purifier unit that includes at least one purifier that removes the residual impurities from unpurified gas supplied from the gas source unit;
a flow split that distributes gas supplied from the gas source unit;
flow mix means for mixing purified gas that has been processed in the purifier unit and unpurified gas supplied from the gas source unit; and
a control unit that determines the purity or impurity content of the gas delivered from the flow mix means and controls the flow split and the flow mix means so that the gas delivered to a demand point has a target purity.

[Fig. 1]

[Fig. 2]

[Fig. 3]

(a)

121a

L1a

CV2

Purified flow
($n_{purified}$, $Q_{purified}$)

L1

PR1

V1

L2

CV1

Output flow
($n_{output}$, $Q_{output}$)

L3

11

Direct flow
($n_{source}$, $Q_{direct}$)

124a

(b)

121a

L1

V2

CV2

11
a

PR1

V1

CV1

124a

L3

11b

[Fig. 4]

[Fig. 5]

(a)

(b)

(c)

[Fig. 6]

(a)

(b)

(c)

[Fig. 7]

(a)

(b)

(c)

Europäisches
Patentamt

European
Patent Office

Office européen
des brevets

# EUROPEAN SEARCH REPORT

**Application Number**

EP 24 20 6248

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 561 506 B1 (DOCZYCK WOLFGANG DIPL-ING [DE]) 23 May 2007 (2007-05-23) | 5 | INV.<br>B01D53/04 |
| Y | * figure 1 *<br>* paragraphs [0001], [0003], [0015] - [0031] * | 1-5 | C07C7/12<br>C07C11/24 |
| | - - - - - | | |
| Y | US 2010/319536 A1 (SONG XUEMEI [US] ET AL) 23 December 2010 (2010-12-23)<br>* figure 3 *<br>* paragraphs [0001], [0080] * | 1-5 | |
| | - - - - - | | |
| Y | WO 2008/120160 A1 (AIR LIQUIDE [FR]; LETESSIER OLIVIER [US] ET AL.) 9 October 2008 (2008-10-09)<br>* figure 1 * | 1-5 | |
| | - - - - - | | |

**TECHNICAL FIELDS SEARCHED (IPC)**

B01D
C07C

The present search report has been drawn up for all claims

| Place of search | Date of completion of the search | Examiner |
|---|---|---|
| Munich | 13 March 2025 | Pöhlmann, Robert |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

  ...............................................................................
& : member of the same patent family, corresponding
   document

EPO FORM 1503 03.82 (P04C01)

**ANNEX TO THE EUROPEAN SEARCH REPORT**
**ON EUROPEAN PATENT APPLICATION NO.**          EP 24 20 6248

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2025

| Patent document cited in search report | | Publication date | Patent family member(s) | | Publication date |
|---|---|---|---|---|---|
| EP 1561506 | B1 | 23-05-2007 | AT | E362801 T1 | 15-06-2007 |
| | | | DE | 102004005626 A1 | 12-01-2006 |
| | | | EP | 1561506 A1 | 10-08-2005 |
| US 2010319536 | A1 | 23-12-2010 | EP | 2272815 A1 | 12-01-2011 |
| | | | KR | 20100138792 A | 31-12-2010 |
| | | | TW | 201107288 A | 01-03-2011 |
| | | | US | 2010319536 A1 | 23-12-2010 |
| WO 2008120160 | A1 | 09-10-2008 | US | 2008242912 A1 | 02-10-2008 |
| | | | WO | 2008120160 A1 | 09-10-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5702540 A
- US 20030168125 A
- WO 2008120160 A
- US 8915992 B
- US 8309473 B
- US 8398747 B